# DEMANDE DE BREVET EUROPEEN

(11) **EP 3 622 918 A1**
(43) Date de publication de la demande: **18.03.2020**
(21) Numéro de dépôt: 19192702.9
(22) Date de dépôt: 15.11.2016
(51) Int. Cl.: A61F 2/24

(54) **ASSEMBLAGE POUR LE REMPLACEMENT DE LA VALVE ATRIO-VENTRICULAIRE TRICUSPIDE**

(30) Priorité: 23.11.2015 FR 1561217
(62) Demande divisionnaire de: 16795096.3
(71) Demandeur: T-Heart SAS, 75012 Paris (FR)
(72) Inventeur: Dibie, Alain, 75015 Paris (FR)
(74) Mandataire: Graf von Stosch Patentanwaltsgesellschaft mbH

(57) **Abrégé**

La présente invention concerne un assemblage pour l'orifice tricuspide d'un cœur humain comprenant une armature extérieure (100) reliée à un stent intérieur (200) portant une bioprothèse (210) de valve tricuspide et une jupe d'étanchéité (610). L'armature extérieure (100) est configurée pour se maintenir dans l'anneau tricuspide natif. Le stent intérieur (200) est relié à l'armature extérieure par un ou plusieurs brins de fixation. La jupe d'étanchéité (610) recouvre l'espace interstitiel existant entre l'armature extérieure (100) et le stent intérieur (200). Des développements sont décrits qui comprennent notamment l'utilisation d'une zone déformable de l'armature, diverses variantes de réalisation de la jupe d'étanchéité, l'utilisation d'une armature constituée de multiples sous-parties, l'utilisation de brins de fixation entre le stent et l'armature et d'éléments de fixation de l'assemblage au tissu natif, l'utilisation de capteurs et/ou d'actionneurs ainsi que l'utilisation d'un stent dans la veine cave inférieure. Des aspects de procédé sont décrits.

## Description

### Domaine de l'invention

L'invention concerne le domaine des procédés et systèmes pour le remplacement d'une valve cardiaque auriculo-ventriculaire défectueuse ou valve *tricuspide.*

### Etat de la Technique

Le cœur humain comporte quatre valves cardiaques. Deux de ces valves sont les valves dites *auriculo-ventriculaires.* La valve *tricuspide* est située entre l'atrium droit (AD) et le ventricule droit (VD). La valve *mitrale* est située entre l'atrium gauche (AG) et le ventricule gauche (VG). Les deux autres valves sont situées entre les ventricules et le système vasculaire. La valve aortique sépare le ventricule gauche de l'aorte et la valve pulmonaire sépare le ventricule droit de l'artère pulmonaire.

Dans certaines situations médicales, il peut être indiqué de réparer ou de remplacer la valve tricuspide. Ces indications médicales sont majoritairement dues à une pathologie fonctionnelle de la valve tricuspide qui peut être fuyante, de façon sévère, secondaire à une dilatation importante de l'anneau tricuspide. Plus rarement cette pathologie est due à une maladie valvulaire rhumatismale ou infectieuse ou bien encore à une dégénérescence d'une bioprothèse sténosante ou fuyante.

De multiples systèmes ont été décrits pour permettre le remplacement de valves cardiaques défectueuses, notamment par voie percutanée ou par voie mini invasive.

Des techniques sont notamment connues pour procéder au remplacement de la valve mitrale. En revanche, très peu de documents scientifiques et *a fortiori* de documents de brevet traitent du remplacement orthotopique de la valve tricuspide.

Les enseignements techniques publiés concernant le remplacement de la valve *mitrale* ne sont pas directement transposables au cas du remplacement de la valve *tricuspide,* notamment pour des raisons physiopathologiques (l'anatomie de l'anneau tricuspide est par nature peu fibreuse, ses dimensions sont supérieures et de forme ovoïde; l'anatomie du ventricule droit est également particulière).

La valve tricuspide est sensiblement plus grande que la valve mitrale. Dans les situations pathologiques, l'anneau tricuspide se dilate dépassant 40 mm de diamètre quand l'anneau pathologique mitral mesure environ 30 à 35 mm. Cette différence entraîne plusieurs conséquences, notamment mécaniques (e.g. maintien, stabilité et en matière de fuites péri-prothétiques).

En revanche, la réciproque est généralement envisageable: les techniques applicables à la valve tricuspide pourraient *a fortiori* l'être à la valve mitrale.

La littérature brevet fait état de documents qui prétendent pouvoir s'appliquer indifféremment aux deux types de valves auriculo-ventriculaires, mais en réalité ces enseignements techniques ne peuvent généralement être appliqués que dans le seul cas de la valve mitrale.

Les documents de brevet US2014/0172070 ou US8657872 sont des exemples de tels documents, pour lesquels les enseignements techniques décrits présentent des limitations et/ou des inconvénients et/ou des insuffisances pour des applications au remplacement de la valve tricuspide.

Bien que proches anatomiquement, la valve aortique et la valve pulmonaire présentent en effet des caractéristiques nécessitant des modes de traitement et/ou de remplacement différents, ne serait-ce que par leur situation anatomique dans le cœur.

En pratique clinique, seule la valve aortique est actuellement remplacée par une valve percutanée de façon courante. Des modèles de bioprothèses pour la valve mitrale percutanée sont actuellement en évaluation clinique. Concernant la valve tricuspide, si les opérations de plastie de la valve et d'annuloplastie (rétrécissement de l'anneau natif) sont connues, les traitements cliniques en matière de bioprothèse orthotopique percutanée sont en cours de développement, à un stade très précoce.

L'invention s'inscrit dans cette dernière évolution.

Il existe un besoin pour des procédés et des systèmes spécifiquement adaptés au remplacement de la valve tricuspide.

### Résumé de l'invention

La présente invention divulgue des systèmes pour le remplacement de la valve auriculo-ventriculaire tricuspide. Selon l'invention, une armature comprend un stent qui lui-même contient la bioprothèse de la valve tricuspide. Le diamètre de l'armature est donc supérieur à celui du stent, porteur de la bioprothèse. Dans un mode de réalisation particulier, le diamètre de l'armature est légèrement supérieur à celui du stent, lui-même adapté au diamètre de la bioprothèse.

La présente invention divulgue également des procédés pour le remplacement de la valve auriculo-ventriculaire tricuspide. La voie est généralement une voie trans-cathéter percutanée. Après un abord percutané, le système contenu dans un cathéter est mis en place par voie vasculaire.

Il est divulgué un assemblage pour orifice tricuspide d'un cœur humain comprenant une armature extérieure reliée à un stent intérieur portant une bioprothèse de valve tricuspide et une jupe d'étanchéité, et dans lequel assemblage l'armature extérieure est adaptée à se maintenir dans l'anneau tricuspide natif (potentiellement et/ou lorsque déployée dans le cœur d'un patient); le stent intérieur est relié à l'armature extérieure par un ou plusieurs brins de fixation; et la jupe d'étanchéité recouvre l'espace interstitiel existant entre l'armature extérieure et le stent intérieur (en conditionnement et/ou lorsque déployée dans le cœur d'un patient).

Dans un développement, la jupe d'étanchéité recouvre en outre l'espace (de contact) existant entre l'armature extérieure et *l'emplacement* du tissu natif (i.e. potentiellement, i.e. dans un état replié ou conditionné) et/ou le tissu natif (i.e. en situation déployée dans le cœur d'un patient) dans la zone de l'anneau.

Dans un mode de réalisation, la jupe d'étanchéité effectue un pli ou un retour, permettant à la fois d'obstruer l'espace de contact avec le tissu natif et de recouvrir l'espace interstitiel entre l'armature et le stent en amont de l'anneau.

Dans un mode de réalisation, la jupe recouvre seulement cet espace interstitiel et d'autres moyens sont utilisés pour empêcher les fuites péri-prothétiques (e.g. bande ou boudin d'étanchéité, colle biocompatible ou matériau de remplissage, etc)

L'armature est précontrainte ou exerce du fait de sa structure une force radiaire (dirigée radialement vers l'extérieur) qui permet de la maintenir dans l'anneau natif. L'armature porte ou comprend ou est associée à un stent, lui-même portant ou comprenant ou étant associé à une bioprothèse. La jupe d'étanchéité permet de canaliser le flux sanguin de l'atrium droit vers le ventricule droit au travers de la bioprothèse (en recouvrant l'espace « interstitiel » (qui est toujours non-nul) existant entre l'armature et le stent portant la bioprothèse d'un diamètre choisi parmi un choix limité). La jupe d'étanchéité permet également de minimiser (ou bien d'éviter entièrement) les fuites péri-prothétiques entre l'armature et le tissu natif (en recouvrant et/ou en bouchant et/ou en obstruant dans la zone de l'anneau l'espace « de contact » existant entre l'armature extérieure plaquée contre le tissu natif et le tissu natif. Le flux sanguin est obstrué (ou stoppé ou bloqué ou obstrué ou empêché) sur toute la circonférence sur la largeur de l'espace « de contact » par la jupe étanche.

Dans un développement, l'armature présente une zone déformable dans la zone correspondant à l'anneau natif

Dans un développement, l'armature recouverte par la jupe d'étanchéité présente en outre en amont de la zone de l'anneau natif une forme renforçant le maintien de l'assemblage dans l'anneau natif.

La forme peut être convexe ou évasée de manière à optimiser la captation du débit sanguin, ce qui contribue à stabiliser ou maintenir le maintien de l'assemblage dans l'anneau tricuspide (une captation appropriée minimise les effets mécaniques de torsion, i.e. de rotation/translation de l'assemblage que la pression du sang pourrait entraîner du fait de la présence de la jupe étanche).

Dans un développement, l'armature présente en outre en aval de la zone de l'anneau natif une forme minimisant la perturbation de l'écoulement du flux sanguin.

De manière générale, la partie de l'armature qui pénètre la zone du ventricule droit est limitée dans ses dimensions (confer les ordres de grandeur donnés ci-après). Dans certains modes de réalisation, la fonction de la forme de cette partie de l'armature peut minimiser les turbulences (e.g. des formes particulières peuvent notamment impliquer des effets de trainée tendant vers un écoulement plus laminaire du flux sanguin)

Dans un développement, l'armature comprend une pluralité de sous-parties.

Dans un mode de réalisation, l'armature est monobloc c'est-à-dire réalisée d'un seul tenant (par exemple, l'armature est imprimée en 3D dans un matériau déformable). Dans un mode de réalisation, l'armature est constituée de deux sous-parties (par exemple complémentaires ou symétriques). Dans un mode de réalisation, l'armature est constituée de trois parties (selon un arrangement économe et robuste). Dans un mode de réalisation, l'armature est constituée de quatre sous parties (arrangement symétrique, avantageux en matière de stabilité). Dans un mode de réalisation particulier, l'armature est constituée d'un grand nombre de sous parties ou fils d'armature.

Dans un mode de réalisation, les différentes sous-parties de l'armature sont solidaires entre elles. Dans un mode de réalisation, les différentes sous-parties de l'armature sont au moins partiellement indépendantes les unes des autres. Dans certains modes de réalisation, au moins une sous-partie de l'armature présente une propriété physique choisie parmi les propriétés comprenant rigidité, élasticité, plasticité, à mémoire de forme, thermosensible, actionnable, instrumentée, configurable et à ressort.

Dans un développement, l'armature extérieure est reliée au stent intérieur par un ou plusieurs brins.

Dans un développement, l'armature comprend quatre sous-parties, chacune des sous-parties étant reliée au stent intérieur par un brin en nitinol.

La configuration à quatre sous-parties et quatre brins rend l'assemblage particulièrement stable versus les effets de torsion mécanique. D'autres modes de réalisation comprennent un ou plusieurs liens « croisés » entre les parties de l'armature entre elles et/ou avec le stent.

Dans un développement, l'assemblage comprend en outre au moins un élément de fixation au tissu natif.

L'élément de fixation au tissu natif améliore notamment le maintien en translation et/ou en rotation de l'assemblage, étant au plus près de la zone de l'anneau natif.

Dans un développement, la disposition de la jupe d'étanchéité est configurable ou reconfigurable ou repositionnable, par exemple au moyen d'un actionneur.

Par exemple, la disposition dans l'espace de la jupe peut être adaptée aux variations de l'espace existant entre le stent de la bioprothèse et l'armature. La jupe peut dans une certaine mesure être actionnée ou repositionnée, par exemple à distance.

Dans un développement, l'armature et/ou le stent et/ou un brin de fixation et/ou un élément de fixation sont constitués d'un matériau thermosensible et/ou à mémoire de forme.

Dans un développement, l'assemblage comprend en outre au moins un capteur et/ou un marqueur.

Le capteur peut notamment être un capteur de position, de déplacement, de pression ou un capteur chimique et/ou biologique (« biomarker »). Le marqueur peut notamment être un marqueur radio-opaque.

Dans un développement, l'assemblage comprend en outre un actionneur adapté modifier la structure de l'assemblage et/ou à ajuster le positionnement d'une partie de l'assemblage par rapport à l'anneau natif.

L'assemblage peut être articulé ou articulable. Le repositionnement peut être relatif (par rapport au tissu natif) et/ou absolu (modification de la forme de la structure de l'assemblage lui-même). Cette reconfiguration peut être manuelle et/ou automatique.

Dans un développement, l'assemblage est relié à un stent adapté à un positionnement dans la veine cave inférieure, ce stent étant relié à l'armature de l'assemblage par un brin de nitinol.

Dans un développement, l'assemblage est replié dans un état conditionné dans un cathéter pour introduction par voie percutanée (e.g. par voie vasculaire ou par voie trans-cardiaque).

Il est divulgué un procédé de remplacement de la valve tricuspide, comprenant des étapes consistant à positionner l'assemblage dans un état replié dans un cathéter, introduire et déployer ledit assemblage par voie percutanée.

Avantageusement, l'espace interstitiel ou « gap » (entre l'armature de diamètre variable et le stent de taille standardisée, c'est-à-dire selon des tailles discrètes et situé à l'intérieur de l'armature) est de dimension variable.

Avantageusement, le stent est fixé à l'armature (par exemple du côté auriculaire) par un ou plusieurs brins, par exemple en nitinol.

Avantageusement, la forme de l'armature comprend trois zones, dont une des zones est située dans la zone de l'anneau natif; l'armature s'applique contre le tissu cardiaque natif de l'anneau tricuspide et tient l'assemblage par sa force radiaire (radiale).

Avantageusement, selon certains modes de réalisation, le stent contenant ou portant la bioprothèse est positionné de manière asymétrique par rapport à l'anneau (majoritairement dans l'auricule et peu présent dans le ventricule droit).

Avantageusement, les intrusions dans le ventricule droit sont minimisées, notamment pour ne pas perturber le flux de la circulation sanguine.

Avantageusement, certains modes de réalisation facultatifs de l'invention comprennent un système de maintien en translation (présence d'un stent dans l'abouchement de la veine cave inférieure, présence d'un ou de plusieurs éléments de fixation sur le tissu natif), ne perturbant notamment pas l'écoulement du flux sanguin.

Avantageusement, différents modes de réalisation de l'invention permettent la mise en place d'une jupe d'étanchéité. La présence de cette jupe d'étanchéité permet notamment de minimiser ou d'éviter les fuites péri-prothétiques entre l'assemblage et le tissu natif. La jupe d'étanchéité peut être en PET (polytéréphtalate d'éthylène) ou dans un autre matériau étanche au sang, couvrant la hauteur de l'anneau et recouvrant la base de l'armature côté auriculaire.

Avantageusement, les modes de réalisation de l'invention permettent de rationaliser la pratique clinique, avec de multiples effets induits avérés ou potentiels (économies d'échelle, standardisation des opérations, sécurité accrue, etc). Les bioprothèses de valve qui sont actuellement commercialisées sont le plus souvent « standardisées », i.e. les différentes gammes de valves existantes sont limitées et les valves sont de dimensions discrètes (généralement 35 mm, 40 mm et 45 mm). Le sur-mesure (de la bioprothèse) est possible, mais onéreux, et présente certains risques inhérents à la fabrication de prothèse non-standard. Les modes de réalisation de l'invention permettent d'éviter à avoir à adapter les dimensions de la valve elle-même aux dimensions exactes de l'anneau tricuspide.

Les dimensions de l'armature d'une part et du stent/bioprothèse d'autre part définissent les dimensions de l'espace interstitiel («gap») existant entre l'armature et le dispositif stent/bioprothèse. Cet espace peut être plus ou moins étendu, généralement de quelques millimètres, selon les configurations et/ou les besoins. Par exemple, le diamètre du stent peut être de l'ordre de 20 % inférieur au diamètre de l'armature au niveau de l'anneau natif (dans cette configuration, la jupe d'étanchéité joue un rôle central pour canaliser le flux sanguin et éliminer les fuites entre le stent et l'armature. Dans un autre cas, le diamètre du stent peut être plus proche du diamètre de l'armature au niveau de l'anneau natif, la jupe d'étanchéité recouvrant l'espacement entre l'armature et le stent.

Plusieurs variables d'ajustement existent selon les modes de réalisation de l'invention, notamment les diamètres de l'armature et le diamètre du stent portant la bioprothèse mesurés au niveau de l'anneau natif. Une variable d'ajustement réside dans les formes et/ou les dimensions de l'armature elle-même. L'armature peut en effet comprendre des géométries (rebonds ou boucles ou rebords ou points d'attache ou ancres ou reliefs, par exemple dans la zone de l'anneau) permettant notamment un ajustement additionnel de l'espace interstitiel existant entre l'armature et le stent/bioprothèse au niveau de l'anneau natif. Une autre variable d'ajustement est le diamètre du stent. Une autre variable d'ajustement est le diamètre de la bioprothèse elle-même.

Avantageusement, le recouvrement par la jupe d'étanchéité pour combler l'espace interstitiel peut lui-même être optimisé (e.g. tension, repli ou retour, jupe comprenant différentes sous-parties, etc). La jupe d'étanchéité entre le stent/bioprothèse et l'armature peut notamment conserver une certaine élasticité (ou tolérance) afin de permettre à l'armature de s'appliquer exactement à la morphologie de l'anneau tricuspide, et ce sans exercer de contrainte.

### Description des figures

D'autres caractéristiques et avantages de l'invention apparaîtront à l'aide de la description qui suit et des figures des dessins annexés dans lesquels:
la figure 1A montre un exemple d'armature selon l'invention ;
la figure 1B illustre un exemple d'interconnexion des sous-parties de l'armature;
la figure 2A représente un stent selon l'état de la technique ;
la figure 2B représente une bioprothèse de valve tricuspide selon l'état de la technique ;
la figure 3A est une illustration du positionnement relatif de l'armature par rapport au stent portant la bioprothèse;
la figure 3B représente une vue en coupe horizontale à la hauteur de la zone de l'anneau ;
la figure 4A montre un exemple d'assemblage selon l'invention;
la figure 4B montre une vue du dessus d'un exemple d'assemblage selon l'invention ;
la figure 5A illustre une variante de réalisation de l'invention;
la figure 5B précise le positionnement anatomique des éléments optionnels de fixation;
la figure 6A illustre l'espacement circonférentiel ou espace interstitiel entre le stent et l'armature;
la figure 6B illustre une variante de réalisation de l'invention comprenant une jupe d'étanchéité ;

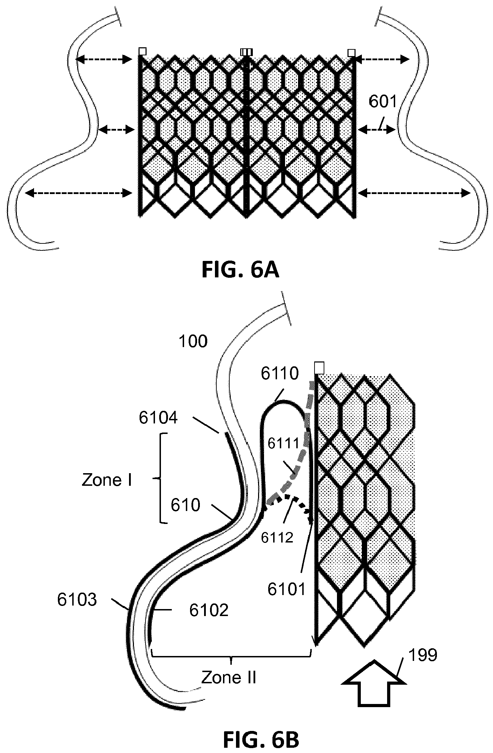

la figure 6B montre une vue en coupe d'un exemple de la jupe d'étanchéité à la hauteur de l'anneau natif ;

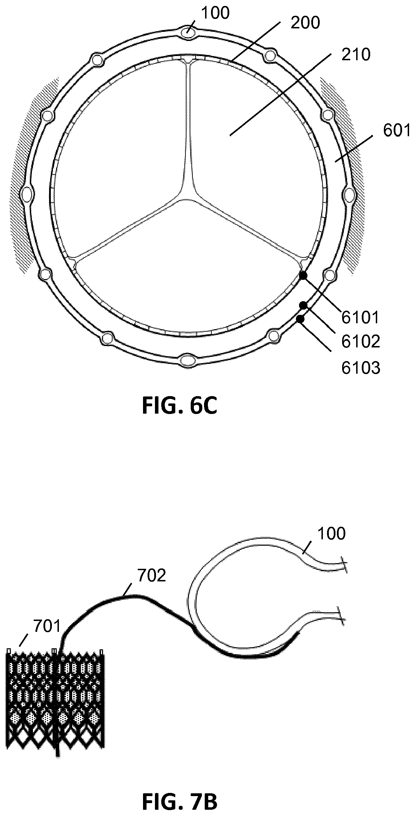

la figure 6C représente une vue en coupe horizontale d'un mode de réalisation de l'assemblage comprenant l'armature, le stent portant la bioprothèse et la jupe d'étanchéité.

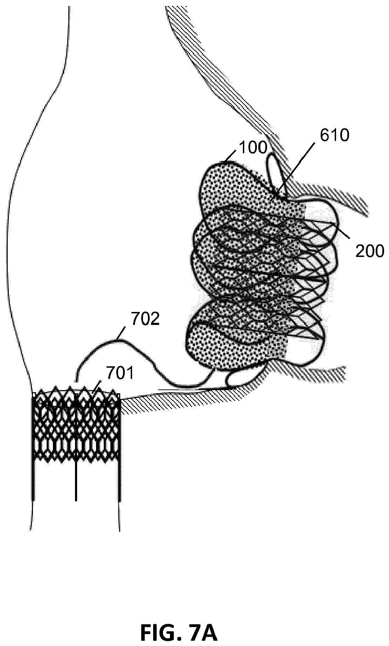

la figure 7A illustre une variante de réalisation de l'invention ;
la figure 7B illustre un mode réalisation optionnel comprenant un stent disposé dans la Veine Cave Inférieure et reliée à l'assemblage par un lien souple de nitinol avec marqueur radio-opaque, fixé à l'armature dans le versant auriculaire AD ;

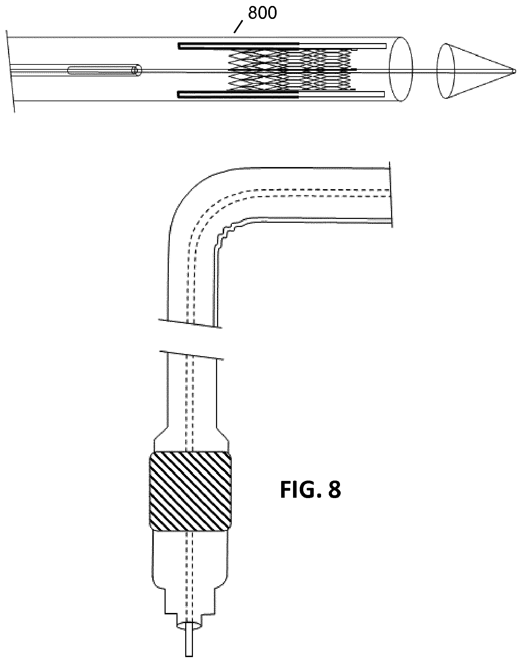

la figure 8 illustre le procédé de mise en place de l'assemblage selon l'invention.

### Description détaillée de l'invention

La valve tricuspide présente des spécificités comparées à la valve mitrale, qui sont détaillées ci-après, notamment en matière de structure propre (e.g. géométrie) et d'environnement de mise en place.

D'un point de vue géométrique, les valves mitrales et tricuspides sont en elles-mêmes très différentes. L'orifice atrio-ventriculaire tricuspide fait communiquer le ventricule droit avec l'atrium homologue droit. Il est muni d'une valve atrio-ventriculaire, formée de trois cuspides, antérieure, septale et postérieure, d'étendue différentes. La valve mitrale, quant à elle, est formée de deux valves ou cuspides (antérieure et postérieure). La valve tricuspide est la plus grande des quatre valves cardiaques, sa surface varie de 5 à 8 cm2 (tandis que la surface de la valve mitrale est comprise entre 4 et 6 cm2). L'anneau de la valve tricuspide présente une forme plus elliptique ou ovoïde que circulaire. Sa structure fibreuse est incomplète, en forme de fer à cheval située dans la zone septale, laquelle est plus solide. En cas d'insuffisance tricuspide, la zone moins solide antérieure (et en partie postérieure) se distend. Son diamètre est variable, de l'ordre de 30 à 32 mm. La valve tricuspide a une circonférence d'environ 120 mm chez l'homme et d'environ 105 mm chez la femme. Elle est formée de trois éléments : le voile valvulaire (les 3 cuspides), l'anneau tricuspide et l'appareil sous-valvulaire (piliers musculaires et de cordages tendineux). Par contraste, la valve mitrale a un diamètre de 28 mm/m2 en diastole. Elle est de forme conique, de l'ordre de 30 mm à l'anneau et de 26mm au sommet des valves (cuspides). Sa circonférence est comprise entre 90 et 100 mm chez la femme, entre 100 et 110mm chez l'homme.

Toujours d'un point de vue géométrique, les environnements des valves mitrales et tricuspides sont très différents. Dans le cœur droit (composé de l'atrium droit et du ventricule droit), l'atrium droit est le confluent du sang veineux chargé en CO2, provenant des deux veines caves. La veine cave inférieure a un diamètre d'environ 30 millimètres (mm). L'atrium droit est plus spacieux que l'atrium gauche, sa capacité (ou volume) est d'environ 160ml, alors que l'atrium gauche est d'un volume d'environ 140ml. La longueur de l'atrium droit est environ 4,5 centimètres, la longueur de longueur de l'OG 3,5 centimètres. Les aspects structurels des ventricules droits et gauches sont contrastés. Le VD a une épaisseur de 5 à 6 millimètres alors que le VG à une épaisseur de 12 à 14 millimètres. La pression moyenne du VD est de 15 millimètres Hg alors qu'elle est de 100 millimètres Hg dans le VG. La pression sanguine est environ cinq fois plus élevée dans le ventricule gauche que dans le ventricule droit.

Le gradient de pression en diastole entre l'AD et le VD est inférieur à 2mmHg. La valve tricuspide correspond à l'orifice atrio-ventriculaire situé dans le système circulatoire fonctionnant à basses pressions.
Le poids du VD est d'environ 70g, le poids du VG est environ 150g. La paroi du VD est donc sensiblement plus fragile que celle du VG. En cas d'implantation d'un système de valve bioprothèse entre l'AD et le VD, il est avantageux de prévoir relativement peu de moyen d'ancrage du côté VD (chambre de remplissage du VD) et de plutôt privilégier la fixation du côté auriculaire AD (en matière de valve mitrale, les ancrages sont généralement placés sous l'anneau mitral côté VG).

Par conséquence, ces différences anatomiques et physiologiques entre la valve mitrale et la valve tricuspide impliquent une configuration mécanique ou de structure significativement différente (notamment en matière de turbulences, de zones de recirculation du flux sanguin, de contraintes et d'efforts mécaniques, de résistance des matériaux et/ou de stabilité des assemblages de remplacement, des procédures chirurgicales de remplacement, etc). Les modes de réalisation prennent en compte et tirent parti de ces différences anatomiques des valves et de leur environnement ; ils sont avantageux notamment en matière de circulation sanguine, de résistance, de stabilité, d'accès, de mise en place et de maintien.

Les dessins fournis sont indicatifs. Par exemple, les formes de l'armature et l'importance de l'espace interstitiel ont parfois été exagérées pour faciliter la compréhension de l'invention, à des fins de lisibilité. En particulier, la forme en crochet de la partie inférieure de l'armature a été exagérée. La fonction de la forme importe seule *in fine,* et il est fait référence aux passages qui détaillent les fonctions visées pour les différentes parties de l'armature.

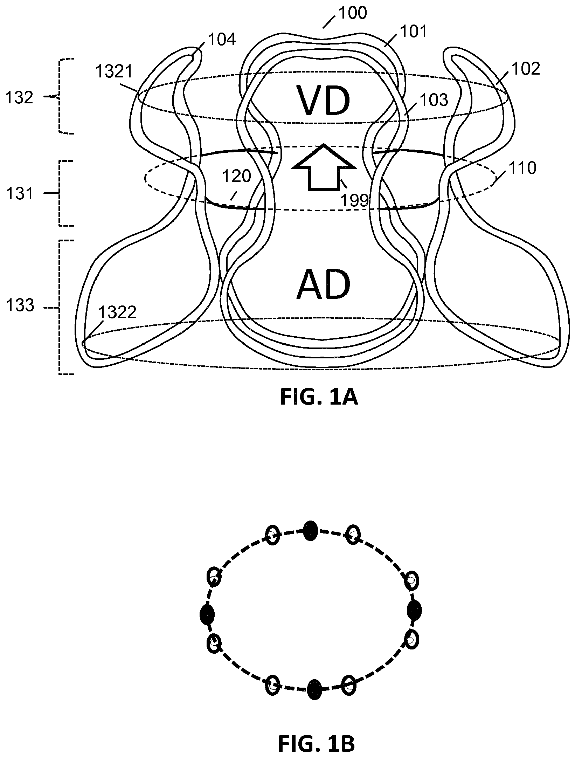

La figure 1A montre un exemple d'armature selon l'invention. Dans l'exemple, l'armature 100 comprend plusieurs parties formées d'un alliage de métaux (par exemple thermosensible ou à mémoire de forme comme un alliage de Nickel-Titane ou nitinol).

Le *nitinol* présente la capacité particulière à changer *d'état physique* du fait d'une variation de température, ce qui en fait un matériau de choix en clinique (conditionné dans un liquide glacé ou rafraichi, le nitinol conserve ensuite sa forme définitive après sa libération dans la circulation sanguine à 37° C)

L'armature est de forme globalement cylindrique présentant trois parties de diamètres différents, lui permettant de se positionner et de se maintenir à travers l'anneau de la valve tricuspide sans entraver le flux sanguin s'écoulant à travers la valve tricuspide. Dans un mode de réalisation, l'armature comprend au moins quatre éléments (101, 102, 103, 104). L'armature présente une hauteur totale comprise entre 32 mm et 45 mm (i.e. adaptée aux dimensions de la bioprothèse). Une bioprothèse 110 est placée à l'intérieur de l'armature

Les quatre parties principales de l'armature 101, 102, 103 et 104 sont reliées entre elles dans la zone de l'anneau valvulaire, qui est la plus étroite, par une jonction ou une articulation souple et déformable. Dans un mode de réalisation, les parties principales sont réalisées en alliage de nickel-titane à mémoire de forme. Chacune des parties principales est schématiquement en forme de S majuscule. L'articulation souple et déformable mesure entre 5 et 7mm dans sa hauteur correspondant à la hauteur de l'anneau valvulaire.

La figure 1B illustre un exemple d'interconnexion des sous-parties de l'armature 100 selon un mode de réalisation. Dans un mode de réalisation, un fil de jonction 110 (unique) relie les différentes parties principales de l'armature, par exemple par des soudures. Dans un mode de réalisation, les parties principales de l'armature sont reliées par une pluralité de jonctions ou articulations (ce dernier mode de réalisation rend la structure selon l'invention plus souple). Avantageusement, les modes de réalisation de ces articulations permettent une adaptation du diamètre de l'armature considérée globalement, en rendant ce diamètre variable et/ou configurable. Dans des variantes de réalisation, le nombre des points de contacts directs peut notamment être ajusté (par exemple augmenté), de manière à accroître la surface de contact avec l'anneau natif et améliorer le maintien de la structure de remplacement de la valve tricuspide. L'exemple représenté à la figure 1B comporte douze points de contact. Ce type de configuration permet avantageusement de réduire le risque de fuite péri-prothétique entre l'armature et le tissu natif de l'anneau tricuspide.

La figure 1A montre que l'armature selon un mode de réalisation de l'invention comprend trois parties ou zones. La direction du flux sanguin est illustrée par la flèche 199.

Dans la zone de l'anneau cardiaque natif 131, l'armature 100 est extensible et/ou déformable. Par sa force radiaire (par exemple précontrainte ou résultant du jeu mécanique des quatre parties interconnectées), l'armature 100 s'adapte à la morphologie de l'anneau tricuspide (qui n'est pas tout à fait circulaire, souvent selon une ellipse). L'anneau natif ou l'armature dans sa partie la plus étroite mesure entre 40 et 42 millimètres. Ce diamètre peut atteindre 45 millimètres, voire plus de 50 millimètres en cas de dilatation importante, correspondant à la zone anatomique de l'anneau tricuspide délimitant le passage ou l'orifice, entre l'AD et le VD. La zone annulaire (de l'anneau tricuspide natif de l'armature selon l'invention) mesure de 5 à 7 mm de hauteur.

Dans la zone intra-VD 132, l'armature se poursuit en aval dans le ventricule droit VD dans le sens du flux sanguin. Dans un mode de réalisation, l'armature s'étend dans le ventricule droit sur une hauteur comprise entre 10 et 12mm, en s'évasant du côté du ventriculaire VD (i.e. présente une forme convexe). Le diamètre maximum 1321, dans la portion intra ventricule droit VD est un peu supérieur au diamètre de la zone de l'anneau. A sa partie distale dans le ventricule droit VD, l'extrémité de l'armature se termine par un diamètre de valeur sensiblement égale au diamètre de la bioprothèse (par exemple de 35mm ou 40mm). Avantageusement, cette configuration mécanique (i.e. forme convexe et sélection de diamètres) permet de maintenir les trois feuillets composant la valve tricuspide native en position ouverte sans créer d'obstacle dans la chambre de remplissage du ventricule droit VD pour l'écoulement du flux sanguin (« *no right ventricular out flow obstruction »* en anglais).

Dans la zone intra-AD 133, située en amont de la zone de l'anneau, se trouve la partie proximale de l'armature, positionnée dans l'atrium droit AD. Dans un mode de réalisation, l'armature présente une forme évasée convexe. Dans un mode de réalisation, le plus grand diamètre 1332 est supérieur de 6 à 8 mm à celui de la zone de l'anneau. Par exemple, la valeur du diamètre peut être de 50 mm si l'anneau mesure 42 mm. L'armature s'étend dans cette zone intra-AD 133 sur une distance variable en fonction des dimensions de la bioprothèse. Par exemple cette distance peut être de l'ordre de 15 mm. Cette zone de l'armature intra AD, correspond à la zone de fixation au stent qui contient la bioprothèse.

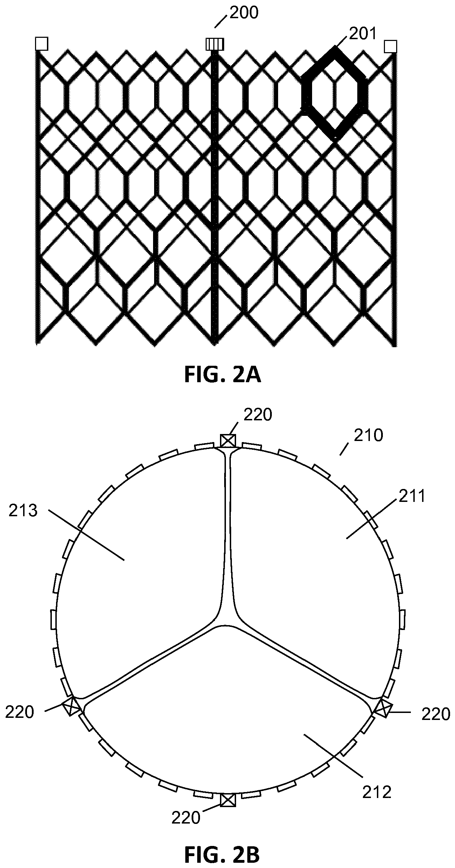

La figure 2A représente un stent 200 selon l'état de la technique. Dans un mode de réalisation de l'invention, le stent 200 utilisé est un stent auto-expandable. Le stent 200 est cylindrique, formé de multiples cellules 201 d'alliage de métaux, par exemple d'un alliage identique à celui de l'armature 100 (titane-nickel). Le diamètre du stent 200 peut être un peu inférieur à celui de l'armature dans la zone de l'anneau. Les dimensions du stent 200 sont généralement discrètes: les dimensions standard sont de 30, 35 et 40 mm. Les dimensions du stent correspondent aux dimensions de la bioprothèse 210 (le stent 200 porte la bioprothèse 210).

La figure 2B représente une bioprothèse 210 de valve tricuspide selon l'état de la technique. Une bioprothèse tricuspide est formée de trois cuspides (211, 212, 213) réalisées à partir d'un tissu animal (par exemple un péricarde bovin ou porcin) et/ou d'un tissu synthétique. Les trois cuspides ou « feuillets » formant la bioprothèse sont reliées (e.g. associées ou fixées ou attachées ou soudées ou cousues) dans ou sur ou par ou via le stent. La bioprothèse fonctionne dans le sens physiologique du flux sanguin arrivant dans l'atrium droit AD et injecté en systole dans la chambre de remplissage du ventricule droit VD.

Différentes technologies peuvent être employées pour relier le stent et la bioprothèse aux différents points de contact 220 (e.g. soudure, colle, lien fixe ou ressort, points de contact flexible ou partiellement rotatif, etc). Dans un mode de réalisation, trois points de contact 220 sont utilisés, permettant une fixation plus sûre de la valve tricuspide. Dans un mode de réalisation, une pluralité de points de contact (supérieur ou égale à quatre) maintient la bioprothèse dans le stent. La probabilité de défaillance décroit généralement lorsque le nombre de points de contact augmente.

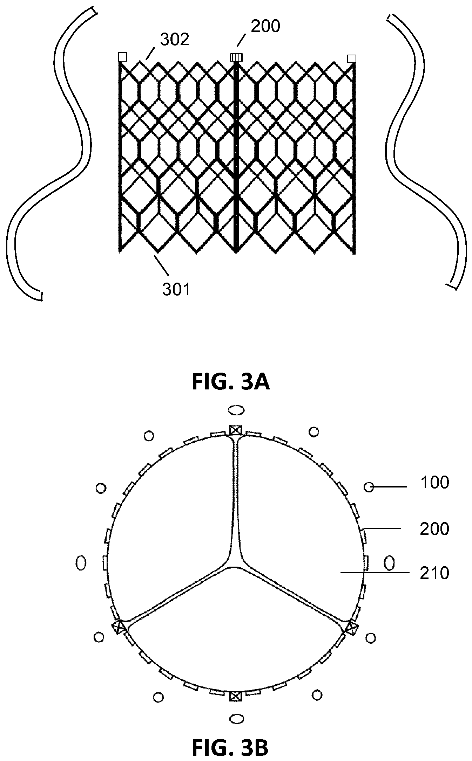

La figure 3A est une illustration du positionnement relatif de l'armature 100 par rapport au stent 200 portant la bioprothèse 210. Dans un mode de réalisation, le stent contenant la bioprothèse est positionnée à l'intérieur de l'armature. L'extrémité proximale 301 (i.e. la base) du stent est située dans la zone AD 133 de l'armature. La bioprothèse 210 est donc majoritairement en position infra-annulaire. L'extrémité distale du stent 302 contenant la bioprothèse 210 est située dans la zone intra-VD 132 mais sur quelques millimètres seulement. Le stent est donc positionné dans une position asymétrique par rapport à la zone de l'anneau, laquelle est la partie la plus étroite de l'armature.

La figure 3B représente une vue en coupe horizontale à la hauteur de la zone de l'anneau. La figure montrant la présence de l'armature 100, du stent 200 et de la bioprothèse 210.

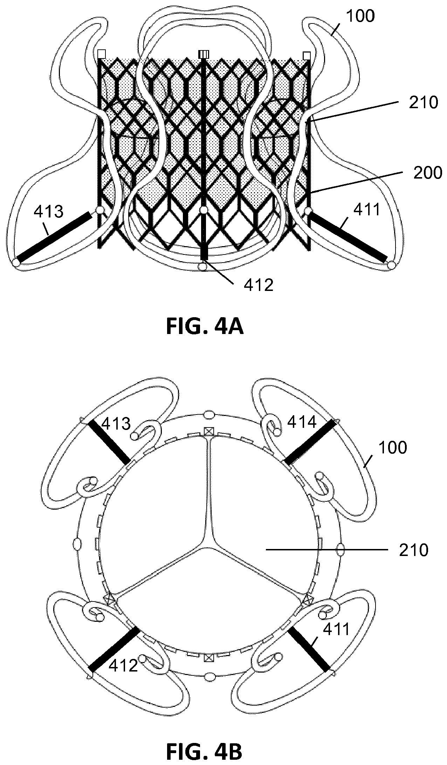

La figure 4A montre un exemple d'assemblage selon l'invention.

L'armature 100 est reliée au stent 200 comprenant la prothèse 210 par un ou plusieurs brins.

Le nombre, l'emplacement et la nature (e.g. matériaux) des brins sont variables selon les modes de réalisation (assemblage plus ou moins flexible, plus ou moins stable ou maintenu, plus ou moins perturbant pour la circulation, etc).

Dans un mode de réalisation particulier, un seul brin de fixation peut être suffisant (la transmission des efforts mécaniques via ce brin unique éventuellement renforcé peut être modélisée et optimisée). Avantageusement, le flux sanguin est perturbé *a minima.*

Des configurations à deux ou trois brins sont possibles.

Dans l'exemple illustré sur la figure, quatre brins 411, 412, 413, 414 maintiennent le stent à l'armature.

Dans un mode de réalisation, les quatre brins de l'exemple représenté sur la figure associent le stent 200 (dans sa zone intra AD 132) à la base de l'armature 100 (dans sa zone la plus large i.e. dans zone intra AD 132). Cette configuration où le rattachement est effectué en amont de la circulation du flux sanguin présente l'avantage d'entraîner une stabilité en torsion du stent en aval. Selon un mode de réalisation (non représenté), en complément ou en substitution du rattachement en amont de la valve et par la base du stent, un ou plusieurs rattachements additionnels et facultatifs du stent à l'armature peuvent être effectués dans la zone de l'anneau, voire par la partie distale du stent (même si ce dernier pénètre peu dans la zone VD).

Dans un mode de réalisation, un ou plusieurs brins (ou « pattes ») sont en nitinol. De manière générale, des matériaux thermosensibles et/ou à mémoire de forme peuvent être utilisés.

Dans un mode de réalisation, un ou plusieurs brins sont rigides. Dans des modes de réalisation, on ou plusieurs brins sont élastiques ou flexibles ou déformables. D'autres modes de réalisation combinent brins élastiques et brins rigides (par exemple en fonction du comportement dynamique de la structure de l'assemblage). D'autres modes de réalisation avancés prévoient l'emploi de brins actionnables ou configurables.

La figure 4B montre une vue du dessus d'un exemple d'assemblage selon l'invention. La figure montre en particulier la mise en place de la bioprothèse 210 au sein de l'armature 100.

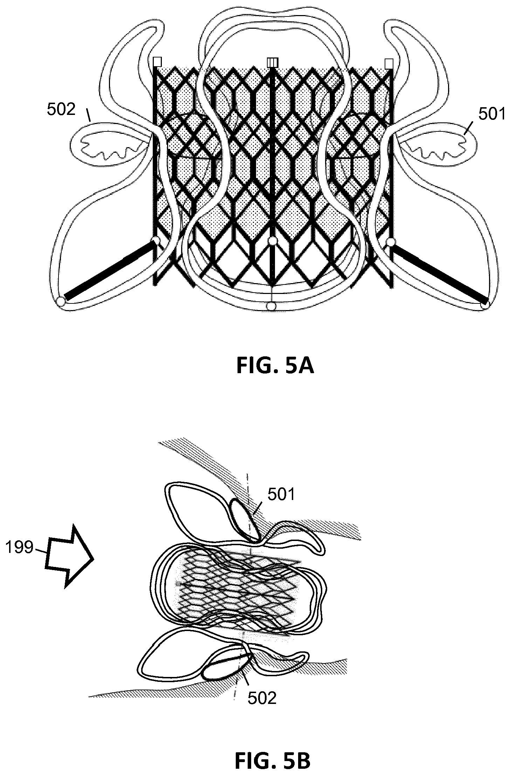

La figure 5A illustre une variante de réalisation de l'invention. L'assemblage comporte un ou plusieurs éléments optionnels de fixation (i.e. de maintien dans le cœur). Dans un mode de réalisation, un élément de fixation revêt la forme d'une « raquette » ou d'une « boucle » ou d'une « patte » (par exemple 501). Dans un mode de réalisation, un élément de fixation est en alliage de nitinol. Un élément de fixation présente une hauteur de l'ordre de 8 à 10mm et une longueur comprise entre 10 et 12 mm. Dans un mode de réalisation, un ou plusieurs de ces éléments optionnels de fixation sont associés à l'armature 100 dans la zone de l'anneau 131 et s'ouvrent uniquement du côté auriculaire AD. Dans certains modes de réalisation de l'invention, aucun élément de fixation n'est utilisé. Dans un mode de réalisation de l'invention, un unique élément de fixation est utilisé. Dans un mode de réalisation de l'invention, deux éléments de fixation sont positionnés de manière symétrique. Pour une stabilité améliorée, un second élément de fixation peut en effet être positionné (par exemple de manière symétrique, e.g. vers la paroi septale inter atriale). Toutefois, cette configuration dans certains cas peut accentuer le risque de trouble de conduction électrique (dont un bloc auriculo-ventriculaire). Dans un mode de réalisation une pluralité d'éléments de fixation est utilisée, diminuant le risque de trouble de conduction.

L'effet technique (la fonction) de tels éléments d'accroche est notamment de stabiliser et de fixer l'armature 100 dans le cœur. En particulier, ces éléments de fixation améliorent la stabilité en rotation et/ou en torsion et/ou en translation de l'assemblage selon l'invention.

La figure 5B précise le positionnement anatomique des éléments optionnels de fixation. Les boucles de maintien sont placées en amont de l'écoulement du flux sanguin 199. Dans le mode de réalisation présenté à la figure 5B, l'assemblage comprend deux éléments de fixation 501 et 502. Ces éléments sont disposés de façon diamétralement opposée par rapport à l'armature. Chaque élément est disposé de manière sensiblement perpendiculaire à l'armature et se plaque sur la paroi du tissu natif. Un des éléments de fixation est positionné du côté du septum inter-auriculaire 501, l'autre élément de fixation 502 est positionné vers la paroi externe de l'AD (dit muscle pectiné). Selon les modes de réalisation, les technologies de rattachement des éléments de fixation à l'armature sont variables. Les points d'attache peuvent par exemple être soudés. Dans une perspective dynamique, la pluralité des éléments de fixation se déploie spontanément ou automatiquement vers l'extérieur au cours de la libération de l'armature lors du retrait du cathéter porteur de l'ensemble du système,

La figure 6A illustre l'espacement circonférentiel ou espace interstitiel entre le stent et l'armature. Dans la zone de l'anneau natif, il existe en effet un espace libre circonférentiel 601, d'amplitude variable entre le stent 200 et l'armature 100. La fonction de l'armature 100 est notamment de maintenir et rigidifier l'ensemble du système tout en appliquant une force radiale contre l'anneau natif pour un placement stable dans le cœur. Le stent et la bioprothèse sont de forme cylindrique (de tailles discrètes et standardisées). Le diamètre du stent et de la bioprothèse sont sensiblement égales, et sont inférieur au diamètre de l'armature : il existe donc un espace interstitiel (un « gap ») entre l'armature 100 et le stent 200.

La figure 6B illustre une variante de réalisation de l'invention comprenant une jupe d'étanchéité (vue en coupe).

De nombreuses variantes de réalisation sont possibles pour le positionnement et la nature de cette jupe d'étanchéité.

La fonction de la jupe d'étanchéité est de rendre étanche (en particulier dans la zone de l'anneau auriculo-ventriculaire) l'assemblage selon l'invention (i.e. le système formé de l'armature et du stent porteur de la bioprothèse); en particulier la jupe d'étanchéité minimise les risques de fuites péri-prothétiques (point 6104 ci-après).

La jupe d'étanchéité 610 prend généralement appui sur l'armature et recouvre au moins partiellement le stent 200.

Dans un mode de réalisation, le point de départ 6101 de la fixation de la jupe d'étanchéité 610 est cousu (ou collé) sur la paroi externe du stent sur une hauteur couvrant largement la zone de l'anneau. Puis, le tissu de la jupe d'étanchéité est plié (par exemple selon un retour à 180° vers l'extérieur) pour redescendre le long de la paroi interne de l'armature 6102. Enfin, le tissu de la jupe d'étanchéité enveloppe par sa base intra-auriculaire l'armature 100 en remontant sur la paroi externe de l'armature 6103 pour se terminer au dessus de la zone de l'anneau, cousue ou collée sur les articulations en S majuscule et/ou les éléments de l'armature 100 renforçant ainsi cette armature dans la zone la plus étroite plaquée contre le tissu natif 6104. Ce point 6104 joue notamment un rôle décisif pour éviter les fuites péri-prothétiques. Concernant les variantes de réalisation, il n'est pas nécessaire que la jupe d'étanchéité enveloppe totalement l'armature.

Dans un mode de réalisation, la jupe d'étanchéité est formée d'une matière synthétique (par exemple du polyéthylène terephtalate ou PET, lequel matériau est souple et étanche à l'eau ou au sang). Dans un mode de réalisation, un matériau étanche comme le dacron peut être avantageusement utilisé. La jupe peut être constituée d'une pluralité de matériaux, par exemple arrangés en couches et/ou en bandes (i.e. avec des zones renforcées par exemple et/ou avec des zones constituées d'une seule couche et/ou des espaces ajourés (des « ouvertures », des « trous », des sous-parties poreuses ou non étanches, etc) en amont de la zone de l'anneau, versant auriculaire, afin de minimiser l'épaisseur de l'assemblage en conditionnement).

La structure de la jupe (e.g. matériaux, arrangement des sous-parties, couches, distribution des espaces ajourés, etc) peut notamment être effectuée de manière à optimiser (i.e. minimiser l'épaisseur de l'assemblage en conditionnement et/ou à assurer la résistance au flux sanguin de la jupe ainsi structurée et/ou à contribuer au renforcement du maintien de l'assemblage mis en place e.g. stabilité en rotation et/ou en translation).

De l'amont vers l'aval, de l'atrium droit vers le ventricule droit, le flux sanguin est légèrement modifié par l'assemblage selon l'invention. En amont, du côté auriculaire, la structure convexe de l'armature implique certaines perturbations de turbulence du flux sanguin, mais cette modification est acceptable ou sans importance médicale et mécanique. Le patient est sous traitement anticoagulant pour éviter la formation de caillots provoqués par la présence de la bioprothèse et de son armature. Par suite, la rhéologie sanguine est modifiée.

Dans la zone I sur la figure 6B (zone ou espace de « contact »), le flux sanguin est « bloqué » (ou stoppé ou bloqué ou obstrué ou empêché) ou au moins substantiellement minimisé dans la zone de l'anneau natif (i.e. sur toute la circonférence et au moins partiellement sur la hauteur du cylindre correspondant de l'anneau valvulaire cardiaque.

Dans la zone II sur la figure 6B (zone ou espace « interstitiel »), la jupe capture le flux sanguin et le canalise au travers de la bioprothèse, du fait de son étanchéité. La forme du repli 6110 (figure 6B) peut incidemment influer sur la recirculation en direction de la bioprothèse. Certains modes de réalisation avantageux consistent à « tendre » ou « relâcher » plus ou moins ce repli de la jupe de manière à optimiser l'écoulement dynamique du flux sanguin. Il existe une légère obstruction au flux sanguin sur le pourtour de la valve elle-même car le diamètre de la bioprothèse est en réalité très légèrement inférieur au diamètre de l'anneau natif. Ce dernier est cependant dilaté par la pathologie justifiant le remplacement de la valve tricuspide, ce qui rend cette obstruction particulière négligeable en proportion. Dans la zone de l'anneau, l'armature est appliquée (en contact) sur le tissu natif. Les fuites entre l'armature et le tissu natif sont en pratique inexistantes ou non significatives (i.e. du fait de l'absence de calcification de l'anneau tricuspide qui ne permet pas l'aménagement d'espaces interstitiels entre l'armature et le tissu natif, et également du fait que le tissu de la jupe effectue un repli dans la zone de l'anneau ce qui améliore l'étanchéité dans cette zone). Une triple épaisseur (de matériau (e.g. de PET) rend étanche le système prothétique, élimine avantageusement les fuites para-prothétiques entre la prothèse et le tissu natif mais peut augmenter l'épaisseur de l'assemblage dans sa configuration repliée. Des variantes de réalisation permettent de concilier ces exigences en déplaçant les zones de repliement ou en agençant les différentes composantes de l'assemblage de manière à optimiser son repliement géométrique.

Certains modes de réalisation de l'invention prévoient l'emploi de brins et/ou de mailles du stent présentant une forme tubulaire ou cylindrique ou elliptique, de façon à minimiser les impacts occasionnés aux globules rouges. Dans un mode de réalisation, les sections des brins et/ou des mailles du stent sont en forme d'ellipse et sont orientées (comme des « volets aéronautiques»), de telle manière à améliorer la rhéologie et/ou optimiser l'écoulement sanguin local et/ou global. Enfin, en aval du côté ventriculaire, le flux est en partie canalisé par le stent sans entraîner de conséquence médicalement préjudiciable (en particulier cela ne créée pas d'obstruction du débit sanguin entrant dans le ventricule droit VD). En aval de l'anneau, l'armature est plus large (forme convexe orientée vers l'extérieur) mais de diamètre inférieur à la zone d'amont, de manière à ne pas gêner la voie d'éjection vers la valve pulmonaire située dans le VD à environ 10mm.

La figure 7A illustre une variante de réalisation entièrement optionnelle de l'invention comprenant une fixation supplémentaire à un stent positionné dans la veine cave inférieure.

Dans certaines situations anatomiques peu fréquentes, il arrive que l'anneau tricuspide - dilaté le plus souvent - ne présente pas de zone d'attache stable ni de calcification. Dans ces cas précis, il est avantageux de mettre en place des dispositifs de fixation spécifiques, additionnels et optionnels, dont la combinaison avec l'assemblage selon l'invention améliore la retenue et le maintien (e.g. en translation) de l'assemblage selon l'invention.

Selon cette variante de réalisation, un stent auto-expandable 701 (par exemple en nitinol) est placé dans la veine cave inférieure VCI, à distance de l'armature. Le stent 701 est de diamètre variable, généralement de l'ordre de 30mm, adapté à la dimension de la VCI à son abouchement dans l'AD. En comparaison des fixations additionnelles prévues pour la valve mitrale, le stent placé dans la VCI doit être moins long de façon à ne pas gêner la circulation dans les veines sus-hépatique. En d'autres termes, l'arrangement de l'arrimage dans le cas de la valve tricuspide est également spécifique lorsqu'on le compare à l'arrangement pour la valve mitrale. Le stent 701 est relié à l'armature 100 de diverses manières (par exemple par un lien filaire 702). Le lien filaire 702 est relié au stent 701 en sa partie haute, au ras de l'abouchement de la VCI. Le lien filaire 702 est relié à l'armature principale à sa base intra AD dans sa partie inférieure. Ce lien filaire 702 peut être rendu visible en fluoroscopie par un marqueur radio-opaque. Ce lien filaire souple et déformable peut être rectiligne ou ondulé. Il peut être adapté dans sa longueur (plus ou moins 30 mm, selon la distance anatomique entre l'anneau tricuspide et la VCI).

La figure 8 illustre le procédé de mise en place du système ou assemblage selon l'invention. Dans un premier état dit « replié » ou « comprimé » ou « conditionné » ou « compressé », le cathéter de pose 800 (« *delivery system »* en anglais) contient l'armature, le stent et la bioprothèse (ainsi que les éléments de fixation et jupe d'étanchéité selon les variantes de réalisation le cas échéant). Dans un second état dit « détendu » ou « libéré » ou « décompressé », l'assemblage selon l'invention se déploie dans le cœur du patient (par manipulation de l'opérateur).

Dans un mode de réalisation de l'invention, la bioprothèse est « repliée » dans le stent, lui-même « replié » (avec la jupe d'étanchéité) dans l'armature dans un état de conditionnement.

Différents modes de réalisation du « largage » de l'assemblage selon l'invention sont décrits ci-après. L'instrument et le dispositif de largage ou «*delivery system»* comprend : a) un guide 0,35 inch de longueur adaptée (longueur proche de 280 mm) recourbé « en escargot » à son extrémité distale non traumatique, prévu pour se placer dans la cavité du VD ; b) un cathéter de longueur adaptée à la distance veine fémorale commune (au creux inguinal) jusqu'à l'AD et le VD. Ce cathéter ou gaine formée d'un tube coaxial dit OTW, est axé sur le guide 0,35.inch. Ce cathéter comporte à sa partie distale une gaine rétractable. Son diamètre de 18 à 24 french est adapté à l'assemblage selon l'invention. La gaine est précédée a son extrémité distale, par un embout conique non traumatique, adapté pour recevoir le guide coaxial 0,35 prévu pour franchir la valve tricuspide et se placer dans la pointe du VD ; c) une poignée qui contient le mécanisme de libération de la bioprothèse. Cette poignée est soudée au tube coaxial. Elle commande le déploiement de l'assemblage. La poignée comporte une molette à vis contrôlant le déploiement et le largage de l'assemblage. Le dispositif est muni également d'un système permettant de couder et orienter le tube à sa distalité, avant la gaine qui contient l'assemblage selon l'invention.

Le procédé de déploiement et de largage de l'assemblage selon l'invention est décrit ci-après. La gaine (18 à 24 Fr) est rétractable et contient l'assemblage selon l'invention (selon ses variantes éventuelles). Dans une première étape, il est procédé à la mise en place percutanée, dans la veine fémorale commune d'un desilet de 24 Fr adapté pour recevoir le système de pose. Dans une seconde étape, le guide 0,35 est placé dans la pointe du VD. Dans une troisième étape, le système de pose, contenant l'armature et la bioprothèse est monté à travers le désilet, puis poussé à travers la veine Cave Inférieure (VCI) jusqu'à l'AD, sur le guide 0,35. Dans une quatrième étape, à l'arrivée dans l'AD, la gaine est coudée a son extrémité vers l'orifice tricuspide a l'aide de la poignée mécanique. Dans une cinquième étape, l'ensemble est ensuite poussé pour franchir la valve tricuspide. Dans une sixième étape, le déploiement progressif de l'armature se fait sous contrôle fluoroscopique (des marqueurs radio-opaques sont situés sur l'armature à la hauteur de la zone de l'anneau) et échocardiographique ETO et/ou 3D. La molette à vis de la poignée tournant dans le sens horaire libère progressivement l'armature contenant la bioprothèse sous contrôle échocardiographique ETO et fluoroscopie. Dans une septième étape, après la zone de l'anneau, dans la partie intra-auriculaire droite AD, le déploiement se poursuit, libérant un ou plusieurs éléments de fixation (« crochets », « raquettes », « boucles ») qui viennent se plaquer contre la paroi externe de l'oreillette et le septum inter auriculaire. Dans le même temps, la bioprothèse est libérée et se met à fonctionner dès que la partie intra AD de l'armature est entièrement libérée. Dans une étape optionnelle correspondant à un mode de réalisation comportant la mise en place d'un stent de fixation VCI, la gaine est retirée progressivement dans l'orifice d'abouchement de la VCI, libérant le stent fixé à l'armature par un brin de nitinol. Cette opération se fait sous contrôle ETO et fluoroscopique et produit de contraste radio-opaque. Finalement, une étape d'angiographie et d'ETO vérifie l'étanchéité, c'est à dire l'absence de fuite de la bioprothèse en place dans la valve tricuspide native.

Dans certains modes de réalisation de l'invention, l'assemblage peut être instrumenté en comprenant des capteurs (« *sensors »* en anglais, par exemple des capteurs actifs de et/ou des marqueurs passifs) et/ou des actionneurs (« *actuators »* en anglais).

L'assemblage selon l'invention peut notamment comprendre des marqueurs radio-opaques permettant de quantifier de mesurer, de vérifier le bon positionnement de l'assemblage dans le cœur du patient.

En complément ou de manière alternative, l'assemblage selon l'invention peut également comprendre des dispositifs permettant des déplacements ou des réajustements dans l'espace de l'assemblage, dont la position peut évoluer voir dériver au cours du temps.

En d'autres termes, l'assemblage selon l'invention peut être statique dans certains modes de réalisation et/ou dynamique ou adaptatif dans d'autres modes de réalisation. L'assemblage selon l'invention peut notamment comprendre un ou plusieurs MEMS. Un microsystème électromécanique ou MEM est un microsystème de dimensions généralement micrométriques comprenant un ou plusieurs éléments mécaniques, utilisant l'électricité comme source d'énergie, en vue de réaliser une fonction de capteur et/ou d'actionneur. Dans des variantes de réalisation, des bio-MEMS sont utilisés. Les actionneurs peuvent être placés dans ou sur différentes parties de l'armature et/ou aux zones d'attaches des différentes sous-parties de l'armature et/ou dans ou sur les brins d'attache de l'armature au stent.

Les actionneurs peuvent notamment, par exemple, servir à reconfigurer la forme de l'armature (par exemple sa convexité) et/ou à ajuster le recouvrement de la jupe d'étanchéité (e.g. courbure, retour, tension de la surface de la jupe à certains endroits, etc) et/ou à ajuster la fixation du stent à l'armature. Les déplacements ou réajustements spatiaux sont généralement effectués sur courtes distances. Ils peuvent être réversibles ou irréversibles (e.g. cliquets mécaniques). Ils peuvent être configurés et/ou configurables. Ils peuvent être au moins partiellement déterminés par un dispositif extérieur (commandés par le médecin opérateur), i.e. en boucle ouverte. Des dispositifs logiques et/ou physiques peuvent sécuriser les modifications spatiales, le cas échéant. Les modifications structurelles apportées à la structure peuvent aussi être régulées selon une boucle fermée, par exemple en fonction des mesures de position statique et de comportement dynamique de l'assemblage inséré dans le flux sanguin.

Dans un mode de réalisation, l'assemblage pour l'invention est modifié de façon à permettre le remplacement de la valve mitrale. Les termes « tricuspide » et « mitrale » ne sont pas interchangeables en général. Dans un mode de réalisation de l'invention particulier, l'assemblage est modifié de façon à permettre le remplacement de la valve mitrale, en tenant compte du fait que le diamètre de la valve mitrale pathologique est significativement moindre que le diamètre de la valve tricuspide pathologique. Entre autres adaptations statiques, comparé à l'assemblage pour la valve tricuspide, il sera donné préférence à l'utilisation, outre d'une bioprothèse mitrale, d'un nombre plus limité de sous-parties composant l'armature (e.g. une stabilité mécanique pouvant être obtenue plus facilement) et si possible un nombre moindre d'épaisseurs de jupe d'étanchéité dans l'état conditionné.

### Articles

1. Assemblage pour l'orifice tricuspide du cœur droit d'un cœur humain comprenant :
   - une armature extérieure (100) reliée à
   - un stent intérieur (200) portant
   - une bioprothèse (210) de valve tricuspide et
   - une jupe d'étanchéité (610)
   dans lequel
   - l'armature extérieure (100) est configurée pour se maintenir dans l'anneau tricuspide natif;
   - le stent intérieur (200) est relié à l'armature extérieure par un ou plusieurs brins de fixation; et
   - la jupe d'étanchéité (610) recouvre l'espace interstitiel existant entre l'armature extérieure (100) et le stent intérieur (200) ; et
   dans lequel la jupe d'étanchéité (610) est configurée pour obstruer l'espace de contact compris entre l'armature extérieure (100) et l'emplacement du tissu natif dans la zone de l'anneau tricuspide natif.
2. Assemblage selon l'une des articles précédentes, dans lequel l'armature extérieure (100) présente une zone déformable configurée pour maintenir l'assemblage à l'intérieur de l'anneau tricuspide natif.
3. Assemblage selon l'une des articles précédentes, dans lequel l'armature extérieure (100) présente en amont de la zone de l'anneau natif une forme configurée pour renforcer le maintien de l'assemblage dans l'anneau tricuspide natif.
4. Assemblage selon l'une des articles précédentes, dans lequel l'armature extérieure (100) présente en aval de la zone de l'anneau natif une forme configurée pour renforcer le maintien de l'assemblage dans l'anneau tricuspide natif.
5. Assemblage selon l'une quelconque des articles précédentes, l'armature extérieure (100) étant reliée au stent intérieur (200) par un ou plusieurs brins (411, 412, 413, 414).
6. Assemblage selon la article 5, dans lequel l'armature extérieure (100) comprend quatre sous-parties (101, 102, 103, 104), chacune des sous-parties étant reliée au stent intérieur par un brin en nitinol.
7. Assemblage selon l'une quelconque des articles précédentes, comprenant en outre au moins un élément de fixation (501, 502) au tissu natif.
8. Assemblage selon l'une quelconque des articles précédentes, la jupe d'étanchéité (610) étant constituée de plusieurs sous-parties et/ou de plusieurs couches de matériaux.
9. Assemblage selon l'une quelconque des articles précédentes, l'armature (100) et/ou le stent (200) et/ou un brin de fixation (411, 412, 413, 414) et/ou un élément de fixation (501, 502) étant constitué d'un matériau thermosensible et/ou à mémoire de forme.
10. Assemblage selon l'une quelconque des articles précédentes, comprenant en outre au moins un capteur et/ou un marqueur radio-opaque.
11. Assemblage selon l'une quelconque des articles précédentes, comprenant en outre un actionneur adapté à modifier la structure de l'assemblage et/ou à ajuster le positionnement d'une partie de l'assemblage par rapport à l'anneau natif.
12. Assemblage selon l'une quelconque des articles précédentes, ledit assemblage étant relié à un stent (701) adapté à un positionnement dans la veine cave inférieure, ledit stent étant relié à l'armature de l'assemblage par un brin en nitinol.
13. Assemblage selon l'une quelconque des articles précédentes, ledit assemblage étant replié dans un état conditionné (800) dans un cathéter pour introduction par voie percutanée.
14. Procédé de remplacement de la valve tricuspide, comprenant les étapes consistant à positionner l'assemblage selon l'une quelconque des articles 1 à 13 dans un état replié dans un cathéter, introduire et déployer ledit assemblage par voie percutanée.

## Revendications

1. Assemblage pour orifice tricuspide d'un cœur humain comprenant :
- une armature extérieure (100) reliée à
- un stent intérieur (200) portant
- une bioprothèse (210) de valve tricuspide et
- une jupe d'étanchéité (610)
dans lequel
- l'armature extérieure (100) est configurée pour se maintenir dans un anneau tricuspide natif
- le stent intérieur (200) est relié à l'armature extérieure par un ou plusieurs brins de fixation ; et
- la jupe d'étanchéité (610) recouvre l'espace interstitiel existant entre l'armature extérieure (100) et le stent intérieur (200) ; et
dans lequel la jupe d'étanchéité (610) est configurée pour obstruer l'espace de contact compris entre l'armature extérieure (100) et l'emplacement du tissu natif dans la zone de l'anneau tricuspide natif.

2. Assemblage selon l'une des revendications précédentes, dans lequel l'armature extérieure (100) présente une zone déformable configurée pour maintenir l'assemblage à l'intérieur de l'anneau tricuspide natif.

3. Assemblage selon l'une des revendications précédentes, dans lequel l'armature extérieure (100) présente en amont de la zone de l'anneau natif une forme configurée pour renforcer le maintien de l'assemblage dans l'anneau tricuspide natif.

4. Assemblage selon l'une des revendications précédentes, dans lequel l'armature extérieure (100) présente en aval de la zone de l'anneau natif une forme configurée pour renforcer le maintien de l'assemblage dans l'anneau tricuspide natif.

5. Assemblage selon l'une quelconque des revendications précédentes, l'armature extérieure (100) étant reliée au stent intérieur (200) par un ou plusieurs brins (411, 412, 413, 414).

6. Assemblage selon l'une quelconque des revendications précédentes, dans lequel l'armature extérieure (100) comprend quatre sous-parties (101, 102, 103, 104), chacune des sous-parties étant reliée au stent intérieur par un brin en nitinol.

7. Assemblage selon l'une quelconque des revendications précédentes, comprenant en outre au moins un élément de fixation (501, 502) au tissu natif.

8. Assemblage selon l'une quelconque des revendications précédentes, la jupe d'étanchéité (610) étant constituée de plusieurs sous-parties et/ou de plusieurs couches de matériaux.

9. Assemblage selon l'une quelconque des revendications précédentes, l'armature (100) et/ou le stent (200) et/ou un brin de fixation (411, 412, 413, 414) et/ou un élément de fixation (501, 502) étant constitué d'un matériau thermosensible et/ou à mémoire de forme.

10. Assemblage selon l'une quelconque des revendications précédentes, comprenant en outre au moins un capteur et/ou un marqueur radio-opaque.

11. Assemblage selon l'une quelconque des revendications précédentes, comprenant en outre un actionneur adapté à modifier la structure de l'assemblage et/ou à ajuster le positionnement d'une partie de l'assemblage par rapport à l'anneau natif.

12. Assemblage selon l'une quelconque des revendications précédentes, ledit assemblage étant relié à un stent (701) adapté à un positionnement dans la veine cave inférieure, ledit stent étant relié à l'armature de l'assemblage par un brin en nitinol.

13. Assemblage selon l'une quelconque des revendications précédentes, ledit assemblage étant replié dans un état conditionné (800) dans un cathéter pour introduction par voie percutanée.

14. Procédé de remplacement de la valve tricuspide, comprenant les étapes consistant à positionner l'assemblage selon l'une quelconque des revendications 1 à 13 dans un état replié dans un cathéter, introduire et déployer ledit assemblage par voie percutanée.
